Europäisches Patentamt

European Patent Office

Office européen des brevets

(19)

(11) **EP 1 097 708 B1**

(12) **EUROPEAN PATENT SPECIFICATION**

(45) Date of publication and mention
of the grant of the patent:
**17.09.2003 Bulletin 2003/38**

(51) Int Cl.7: **A61K 31/20**, A23L 1/30,
A61P 29/00

(21) Application number: **00203510.3**

(22) Date of filing: **11.10.2000**

(54) **Use of trans-trans isomers of conjugated linoleic acid**

Verwendung von trans-trans-Isomeren von konjugierter Linolsäure

Utilisation d'isomères trans-trans de l'acide linoéique conjugé

(84) Designated Contracting States:
**AT BE CH CY DE DK ES FI FR GB GR IE IT LI LU
MC NL PT SE**

(30) Priority: **02.11.1999 EP 99308714**

(43) Date of publication of application:
**09.05.2001 Bulletin 2001/19**

(73) Proprietor: **Loders Croklaan B.V.
1521 AZ Wormerveer (NL)**

(72) Inventors:
• **Rogers, Julia Sarah
Sharnbrook, Bedford MK44 1LQ (GB)**
• **Barclay, Scott S.
Sharnbrook, Bedford MK44 1LQ (GB)**
• **Parmar, Preyesh
Sharnbrook, Bedford MK44 1LQ (GB)**
• **Cain, Frederick William
1521 AX Wormerveer (NL)**
• **Taran, Victoria
1521 AX Wormerveer (NL)**

(74) Representative: **Stevens, Ian Edward et al
Eric Potter Clarkson,
Park View House,
58 The Ropewalk
Nottingham NG1 5DD (GB)**

(56) References cited:
WO-A-01/05395          WO-A-97/18320
US-A- 4 118 342        US-A- 4 164 505
US-A- 5 208 356

• **DATABASE BIOSIS [Online] BIOSCIENCES
INFORMATION SERVICE, PHILADELPHIA, PA,
US; PREV198273063183, 1981 KINSELLA J.E. ET
AL.: "Metabolism with trans fatty acids with
emphasis on the effects of trans trans
octadecadienoate on lipid composition,
essential fatty-acid and prostaglandins"
XP002139503 & AMERICAN JOURNAL OF
CLINICAL NUTRITION, vol. 34, pages 2307-2318,**
• **DATABASE BIOSIS [Online] BIOSCIENCES
INFORMATION SERVICE, PHILADELPHIA, PA,
US; PREV198069057081, 1979 KINSELLA J.E.
ETAL.: "Prostaglandins and their precursors in
tissues from rats fed on trans trans linoleate"
XP002139504 & BIOCHEMICAL JOURNAL, vol.
184, - 1979 pages 701-704,**

EP 1 097 708 B1

**Description**

Conjugated linoleic acid is indicated in many literature references as a composition having a number of health benefits. In particular WARF filed many patent applications on these benefits.

Conjugated linoleic acid however is not a single compound, but consists of a great number of isomers. These isomers include isomers wherein the 2 double bonds have different positions in the fatty acid molecule, but also isomers based on cis/trans isomerism.

So far all references on conjugated linoleic acid strongly suggest that the health effects that are reported are due to the presence of at least a cis double bond in the system. In particular the $cis^9trans^{11}$ and $trans^{10}cis^{12}$ isomers are held responsible for the beneficial effects. This can be concluded from eg. the following documents: WO 99/29317; WO 96/34855 but also from scientific publications such as, Poultry Science 72 (1993) p.1301-1305 or J Lipid Research 40 (1999) p.1426 - 33 or Lipids 33 (5) 1998, p.521-7. In contrast, Lipids 34 (3), 1999, p.235 -41, shows that $trans^9trans^{11}$ CLA has no biological effect at all.

We studied whether these effects really are due to the isomers mentioned above or whether other isomers from CLA either contribute to these known effects or display novel effects. This study resulted in the surprising finding that in in-vitro tests, in particular the tt-CLA compounds (ie conjugated linoleic acid isomers wherein all bonds are trans double bonds) have beneficial properties, in particular anti-inflammatory properties. Moreover we found that a tt CLA isomer mixture containing predominantly $trans^9trans^{11}$ and $trans^{10}trans^{12}$ isomers displayed the strongest biological effect.

Therefore our invention concerns in the first instance the use of a composition for the production of a food product or food supplement wherein a composition comprising 1 to 100, preferably 5 to 90, most preferably 20 to 85 wt % (based on total composition) of isomers of trans/trans conjugated linoleic acid (=tt-CLA) as measured by $^{13}$C-magnetic resonance, wherein the tt-CLA is applied for the production of a food product or food supplement with anti-inflammator properties.

According to another embodiment of our invention these compositions can be applied as an additive in a food product or as a food supplement, preferably in encapsulated form in order to provide these products with anti-inflammatory properties. The use of these tt-rich CLA compositions simultaneously was found to result in an improvement of the physical properties, such as improved hardness, texture, firmness and overrun of food products.

The mouthfeel, oral meltdown and flavour release are not negatively affected by the tt-CLA. In addition, the products are easier to process and have better aeration properties.

It is preferred that the tt-CLA compositions according to the invention have a high relative tt-CLA content. Therefore we prefer to use a composition, that comprises the tt-CLA isomers as measured by $^{13}$C magnetic resonance techniques in amounts of 5 to 90 wt% (on total composition), while the CLA isomers $cis^9trans^{11}$ and $trans^{10}cis^{12}$ are present in a weight ratio of: $(t^9t^{11}+t^{10}t^{12}):(c^9 t^{11} +t^{10} c^{12})$ of more than 3:1, preferably more than 5:1, most preferably more than 7.5:1.

The $^{13}$C magnetic resonance technique for the determination 5 of the isomer distribution in the CLA is known from Davis, A.L. et al Chemistry and physics of Lipids, 97, (1999) p.155-65.

The tt-rich CLA mixtures that can be applied according to the invention can be selected from free fatty acids, mono-, di- or triglycerides and alkylesters from CLA.

Although tt-CLA always will be produced in some (minor) amounts during the production of CLA isomer mixtures it would be very beneficial if concentrates would be available, as these would ease the dosing into foods and in particular would enable to make effective food supplements. Therefore we studied whether we could obtain concentrates of tt-CLA. This study resulted in another embodiment of our invention ie. in compositions with anti-inflammatory properties comprising CLA isomers, wherein the composition comprises more than 10 wt%, preferably more than 25 wt%, most preferably more than 50 wt% of CLA-isomers from which more than 55, preferably more than 60, most preferably more than 65 wt% (on total of CLA isomers) are tt-CLA isomers as measured by $^{13}$C-magnetic resonance techniques.

Preferred compositions that we obtained are compositions, wherein the tt-CLA isomers $t^9t^{11}$ and $t^{10}t^{12}$ are present in a weight ratio $t^9t^{11}:t^{10}t^{12}$ of less than 2.3:1, preferably less than 1.8:1, most preferably less than 1.0:1 (as measured by $^{13}$C magnetic resonance techniques).

Other preferred compositions comprise in addition to the tt-isomers $c^9t^{11}$-and $t^{10}c^{12}$-CLA isomers in a weight ratio of (total $t^9t^{11} + t^{10}t^{12}):(c^9t^{11} + t^{10}c^{12})$ of more than 3:1, preferably more than 5:1, most preferably more than 7.5:1 as measured by $^{13}$C-magnetic resonance technology.

The tt-CLA composition according to the invention can suitably be applied in food products. Although the tt-CLA compositions could be present in any form (ie free acid / glycerides / alkylesters with alkyl groups with 2-20, preferably 2-8 carbon atoms) we prefer to use these compositions as glycerides because in that way the best oral mouthfeel properties of the food can be achieved.

Part of our invention therefore also are food products, preferably selected from the group consisting of confectionery products, spreads, sauces, dressings, mayonnaise, cheese, ice cream, doughs, baked bakery products, fillings and creams containing an effective amount of a composition comprising 1 to 100 wt% of tt-CLA, preferably in the form of a mono- and/or di- and/or triglyceride. Most preferred are food products containing an effective amount of the preferred CLA-composition as defined above. An effective amount is defined as that amount, that corresponds with the recommended daily amount as achievable in 1-5 foodservices per day.

A very convenient method to administer an effective dose of our tt-CLA to the users is by providing our users with food supplements containing an effective dose of the tt-CLA. Therefore part of our invention is also food supplements, wherein the tt-rich CLA is encapsulated in a food grade encapsulating material, such as sugar, lactose, starch, modified starch, gelatine, cyclodextrin, proteins and cellulose.

The food supplements can also be fortified with other food ingredients. These ingredients can be selected from the group consisting of vitamins A, B, C, D, E, K, minerals such as calcium, potassium, magnesium, iron, copper, zinc, selenium and anti-oxidants such as tocopherols, polyphenols.

The tt-rich CLA isomer composition can be made by a process

i) wherein a composition with a total unconjugated C18:2 content of more than 55 wt% is conjugated and isomerized subjecting it to a catalytic treatment with a Sulphur-Nickel catalyst at a temperature of 100 to 180 °C in the absence of hydrogen

ii) the product formed in step i) is subjected to a solvent fractionation using acetone in a weight ratio of acetone to CLA product of 10:1 to 1:1 at a temperature of -25 to -100 °C, whereupon a stearine (or top) fraction is isolated as product according to the invention.

An alternative process for making these compositions comprises an enrichment of a mixture comprising different isomers of conjugated linoleic acid in the tt-CLA isomers wherein a mixture comprising at least 1 wt% of tt-CLA in admixture with other CLA isomers is subjected to an enzymic conversion, selected from the group consisting of i) partial hydrolysis of a glyceride mixture or of a mixture of esters of short chain alcohols, ii) partial esterification of a mixture of free fatty acids with glycerol or with a glyceride, iii) partial glycerolysis of a glyceride mixture and iv) partial esterification of an alcohol with free CLA isomers, while using an enzyme that can discriminate tt-CLA from other CLA isomers, whereupon the reaction product is separated by physical means separating a fraction that is enriched in tt-CLA as reaction product.

Enzymes that can be applied in above process can be selected from the group consisting of Candida rugosa lipase; Lipase QL; Lipase SL, Lipase OF and Geotrichum candidum B lipase, Lypozyme IM, Lipozyme M.

## EXPERIMENTAL PART

The anti-inflammatory effects were determined by in *vitro* tests wherein the production Prostaglandin E2 (=PGE2) by the human skin fibroblasts, blood vessel endothelial cells (HUVECS=Human Umbilicial Vein Endothelial Cells) and blood is measured following stimulation by the inflammatory modulus PMA. A reduction of the levels of PGE2 is indicative for the anti-inflammatory effect.

Primary human foreskin fibroblasts at passage 2 (P2) were seeded into 96-well plates at 10000 cells/well and maintained for 24 hours in an atmosphere of 5% carbon dioxide in Dulbeccos Modified Eagles Medium (DMEM) 5 supplemented with 10% foetal calf serum. Enriched tt-CLA (containing 84 wt% of tt-CLA and having a ratio ($t^9t^{11}+t^{10}t^{12}$): ($c^9t^{11}+t^{10}c^{12}$) of 15 was added to fresh cell media in ethanol (final concentration 1%) in triplicate and incubated for a further 24 hours. Phorbal myristate acetate (PMA, Sigma) in ethanol/cell media was added to the media (final concentration 10nm) and the cells incubated for a futher 24 hours. PMA represents an external stressor which induces oxidative stress and inflammatory responses in cells. The media was then analysed immediately as described below.

**Prostaglandin E2 (PGE2) assay** Volumes of 50 µl culture medium were taken for PGE2 assay after gently shaking the culture plate. PGE2 levels in the medium were determined with a Biotrak PGE2 immunoassay kit (Amersham, UK). The assay is based on the competition between unlabelled PGE2 in the sample and a fixed quantity of horseradish peroxidase labelled PGE2 for a limited amount of fixed PGE2 specific antibody. Concentrations of unlabelled sample PGE2 were determined according to a standard curve which was obtained at the same time.

Results:

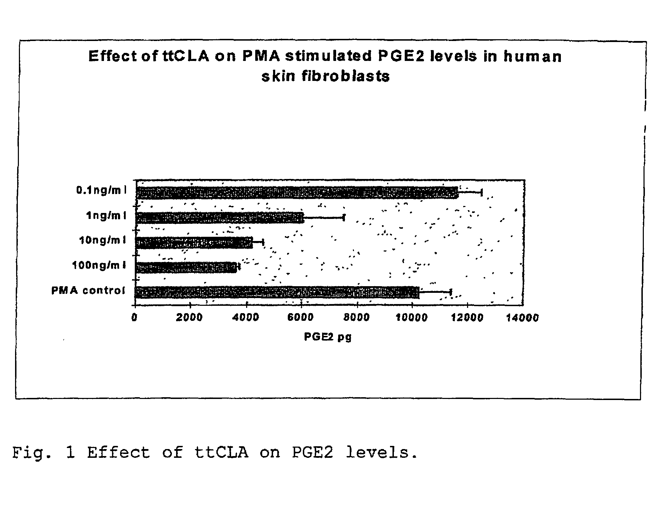

The below graph (Fig 1) demonstrates that challenging cells with an inflammatory stimulus such as PMA (Phorbol myristyl acetate) causes an increase in the inflammatory response as measured by prostaglandin E2 (PGE2) production. ttCLA, even at the levels of lOng/ml, dramatically reduces the inflammatory response as measured by PGE2 production (Fig. 1).

- good anti-inflammatory activity.

Example 1

10g of sunflower oil were mixed with 1g of sulphur poisoned nickel hydrogenation catalyst. The mixture was stirred under a blanket of nitrogen at 180 °C in a glass vessel equipped with a magnetic stirrer.
After 6 hours a sample was removed extracted with a suitable solvent and filtered. The fatty acid composition of the triglyceride, as determined by FAME GC, contained 45% conjugated linoleic acid (CLA) of which 50% was tt CLA. The major tt isomers, determined by $^{13}$C NMR, were trans$^9$trans$^{11}$ and trans$^{10}$trans$^{12}$ which together represented 60% of the total tt CLA.

Example 2

Conjugated linoleic acid was produced as described in example 1. An enriched product was made according to the following procedure. 5g of CLA was added to 30g of acetone in a stirred glass vessel and the temperature slowly reduced to -58 °C. The stearine fraction was isolated by vacuum filtration and washed with pre-cooled acetone. The fatty acid composition, determined by 13C-NMR, contained 33% CLA of which 86.6% was tt CLA. The major tt isomers, determined by $^{13}$C NMR, were trans$^9$trans$^{11}$ and trans$^{10}$trans$^{12}$ which together represented 67% of the total tt CLA. This product was tested in above test on anti-inflammatory properties.

Example 3

Conjugated linoleic acid was produced by alkali isomerisation as described previously, WO9718320, to give a product that contained 80% CLA. The two main isomers, determined by FAME GC, were cis$^9$trans$^{11}$ and trans $^{10}$cis$^{12}$ which together represented 95% of the total CLA.
20g of this material was heated to 250 °C for 4 hours under a blanket of nitrogen. The fatty acid composition, determined by FAME GC, contained 67% CLA of which 30% was tt.

Example 5

Conjugated linoleic acid was produced as described in example 1. An enriched product was made by adding 2 g of this CLA to 2 g of water and 3,000 LU of lipase B from Geotrichum candidum. The mixture was stirred and maintained at 35 oC. The fatty acid composition of the glycerides as determined by FAME GC contained 50 wt% of CLA of which 57 % was tt CLA.
**Definitions for products used in examples 6 and 7**

InES: Interesterified palm oil stearin/palmkernel stearin fat
Pof IV65 : Palm olein fraction with Iodine Value 65
SF : Sunflower oil
tt CLA : tt CLA containing oil
CN : Coconut oil

Example 6 Preparation of margarine

**a) Formulation**

| Aqueous Phase | |
|---|---|
| Water | 18.48% |
| Potassium Sorbate | 0.15 |
| Citric Acid | 0.07 |
| SMP | 1.0 |
| Fat Phase | |
| Fat Blend | 80.0 |

**4**

(continued)

| Fat Phase | |
|---|---|
| Hymono 8903 | 0.3 |

Fat Phase: -Product 1. 12% InEs, 88% SF (Control)
        Product 2. 12% InEs, 10% tt CLA, 78% SF

**b) Process Conditions**

The process line was configured as: -

Premix - Pump - $A_1$-unit - $C_1$-unit - $A_2$-unit

Premix temperature was set at 60°C and 60-rpm stirrer speed. All units were set to 15°C, with shaft speeds set to 1000 rpm. Throughput was 50 g/min. using the constant displacement pump.
For all products a coarse premix was prepared by slowly adding the prepared aqueous phase to the oil phase in the premix tank. A 2 kg-batch was employed.
The mix was stirred for 15 minutes before pumping. After pumping the line was allowed to run for 15 minutes before any collection of product.
The following process parameters were noted: -

| Product | $A_1$ exit (°C) | $C_1$ exit (°C) | $A_2$ exit (°C) | Line Pressure (bar) |
|---|---|---|---|---|
| Control | 20.2 | 19.4 | 17.6 | 1.0 |
| 10% tt CLA | 21.4 | 20.2 | 18.0 | 1.4 |

All tubs were placed at 5°C. After one day, one tub of each was transferred to each of 5°, 10°, 15° and 20°C for evaluations after one week.
All samples spread easily with no apparent water loss. All products are of excellent quality and displayed very good values for hardness (C-value), collar and conductivity at all storage temperatures (5, 10, 15 and 20°C).

5°C Storage

| Sample | C-Value (g/cm$^2$) | Collar (Scale I to VI) | Conductivity ($\mu$Scm$^{-1}$) |
|---|---|---|---|
| Control | 630 | I/II | $<10^{-5}$ |
| 10% tt CLA | 610 | I/II | $<10^{-5}$ |

10° Storage

| Sample | C-Value (g/cm$^2$) | Collar (Scale I to VI) | Conductivity ($\mu$Scm$^{-1}$) |
|---|---|---|---|
| Control | 410 | I | $<10^{-5}$ |
| 10% tt CLA | 370 | I | $<10^{-5}$ |

15°C Storage

| Sample | C-Value (g/cm$^2$) | Collar (Scale I to VI) | Conductivity ($\mu$Scm$^{-1}$) |
|---|---|---|---|
| Control | 340 | I | <10$^{-5}$ |
| 10% tt CLA | 390 | I | <10$^{-5}$ |

20°C Storage

| Sample | C-Value (g/cm$^2$) | Collar (Scale I to VI) | Conductivity ($\mu$Scm$^{-1}$) |
|---|---|---|---|
| Control | 300 | I | <10$^{-5}$ |
| 10% tt CLA | 280 | I | <10$^{-5}$ |

Example 7 Preparation of ice cream

Table 1:

| Recipe | |
|---|---|
| The following recipe was applied (cf table 1) | |
| Component | Wt% |
| Fat blend | 10.0 |
| Skimmed milk powder | 10.0 |
| Crystal sugar | 12.0 |
| Clear syrup | 4.0 |
| Dextrose anhydrate | 2 |
| Dimodan PVP | 0.6 |
| Water | 61.4 |

The fat blends that were used are disclosed in table 2

Table 2:

| Fat blend | | |
|---|---|---|
| | Component | Wt% |
| Reference | POf IV65/ CN/ SF | 30/20/50 |
| Sample | POf IV65/CN/CLA tt containing oil | 30/20/50 |

The sugar, milk powder and dextrose were mixed and added to the water. The mixture was heated to 70°C and the clear syrup was added. Next the fat blend and the emulsifier were added. The emulsion was stirred with an ultra-turrax, cooled down to 20°C and stirred again with the ultra-turrax. The emulsion stayed overnight in the refrigerator at 7°C. The batch ice cream machine was held for 24 hours at -28°C. The emulsion was stirred in the machine for 40 minutes until the temperature was at its lowest. The resulting ice cream was stored at -18°C for at least 3 days and was then evaluated.

From table 3 it can be concluded that the products according to the invention displayed a better hardness and a higher overrun.

Table 3:

|  | Reference | Product with CLA t,t |
|---|---|---|
| Overrun in % | 6.7 | 14.4 |
| hardness | 52 | 173 |

**Taste panel**

Seven panellist tasted the firmness of sample in comparison to the reference. For 5 out of seven the sample with CLA t,t had a higher firmness.

1. Reference with SF - CLA tt

|  | less/slower | equal | more/faster |
|---|---|---|---|
| Firmness |  | 2 | 5 |

**Claims**

**1.**   Use of a composition for the production of a food product or a food supplement wherein a composition comprising 1 to 100, preferably 5 to 90, most preferably 20 to 85 wt% (on total composition) of isomers of trans/trans conjugated linoleic acid (=tt-CLA) as measured by $^{13}$C-magnetic resonance is applied for the production of a food product or a food supplement with anti-inflammatory properties.

**2.**   Use of a composition according to claim 1 wherein the composition, comprising the tt-CLA is applied for the production of a food supplement in encapsulated form.

**3.**   Use of a composition according to claims 1 and 2 wherein the composition, comprising the tt-CLA isomers contains as measured by $^{13}$C magnetic resonance techniques 5 to 90 wt% (on total composition) of tt-CLA, while also the CLA isomers cis$^9$ trans$^{11}$ and trans$^{10}$ cis$^{12}$ are present in a weight ratio of (total t$^9$t$^{11}$+t$^{10}$t$^{12}$) to (c$^9$t$^{11}$+t$^{10}$c$^{12}$) of more than 3:1, preferably more than 5:1, most preferably more than 7.5:1.

**4.**   Use of a composition according to claims 1 to 3 wherein the tt-CLA isomers are applied either as free fatty acids, or as mono-, di-, and/or triglycerides or as alkylesters.

**5.**   Composition with anti-inflammatory properties comprising CLA isomers, wherein the composition comprises more than 10 wt%, preferably more than 25 wt%, most preferably more than 50 wt% of CLA-isomers, from which more than 55, preferably more than 60, most preferably more than 65 wt% (on total CLA isomers) are tt-CLA isomers as measured by $^{13}$C-magnetic resonance techniques.

**6.**   Composition with anti inflammatory properties according to claim 5, wherein the composition comprises the tt-CLA isomers t$^9$t$^{11}$ and t$^{10}$t$^{12}$ in a weight ratio t$^9$t$^{11}$ to t$^{10}$t$^{12}$ of less than 2.3:1, preferably less than 1.8:1, most preferably less than 1.0:1 (as measures by 13C magnetic resonance techniques).

**7.**   Composition according to claims 5 or 6 wherein the composition also contains c$^9$t$^{11}$ and t$^{10}$c$^{12}$-CLA isomers in a weight ratio of (total t$^9$t$^{11}$+t$^{10}$t$^{12}$) to (c$^9$t$^{11}$+t$^{10}$c$^{12}$) of more than 3:1, preferable more than 5:1, most preferable more than 7.5:1 as measured by $^{13}$C-magnetic resonance technology.

**8.**   Food products, preferably selected from the group consisting of confectionery products, spreads, sauces, dressings, mayonnaise, ice cream, doughs, baked bakery products, fillings, creams containing an effective amount of a composition comprising 1 to 100 wt% of tt-CLA, preferably in the form of a mono- and/or di- and/or triglyceride and most preferably containing an effective amount of the composition according to claims 5 to 7.

**9.**   Food supplements containing an effective amount of a composition comprising 1 to 100 wt% of tt-CLA, preferably containing an effective amount of a composition according to claims 5 to 7.

10. Food supplements according to claim 9 wherein the tt-CLA rich composition is encapsulated in a food grade encapsulating material.

11. Food supplement according to claims 9 to 10 wherein the composition comprising the tt-CLA is fortified with other food ingredients selected from the group consisting of vitamins A, B, C, D, E, K, minerals such as calcium, potassium, magnesium, iron, copper, zinc, selenium and anti-oxidants such as tocopherols, polyphenols.

12. Process for the preparation of a composition according to claims 5 to 7, wherein:

   i) a composition with a total unconjugated C18:2 content of more than 55 wt% is conjugated and isomerized by subjecting it to a catalytic treatment with a Sulphur-Nickel catalyst at a temperature of 100 to 180 oC in the absence of hydrogen

   ii) the product formed in step i) is subjected to a solvent fractionation using acetone in a weight ratio of acetone to CLA product of 10:1 to 1:1 at a temperature of -25 to -100 °C, whereupon a stearine (or top) fraction is isolated as product according to claims 5 to 6.

13. Process for the enrichment of a mixture comprising different isomers of conjugated linoleic acid in the tt-CLA isomers wherein a mixture comprising at least 1 wt% of tt-CLA in admixture with other CLA isomers is subjected to an enzymic conversion, selected from the group consisting of i) partial hydrolysis of a glyceride mixture or of a mixture of esters of short chain alcohols, ii) partial esterification of a mixture of free fatty acids with glycerol or with a glyceride, iii) partial glycerolysis of a glyceride mixture and iv) partial esterification of an alcohol with free CLA isomers, while using an enzyme that can discriminate tt-CLA from other CLA isomers, whereupon the reaction product is separated by physical means separating a fraction that is enriched in tt-CLA as reaction product.

14. Process according to claim 13 wherein the enzyme is selected from the group consisting of: Candida rugosa lipase, Lipase QL, Lipase SL, Lipase OF, Geotrichum candidum B lipase, Lypozyme IM and lypozyme M.

**Patentansprüche**

1. Verwendung einer Mischung zur Herstellung eines Nahrungsmittels oder eines Nahrungsmittelzusatzes, worin eine Mischung, die, gemessen mit $^{13}$C-Magnetresonanz, 1 bis 100, vorzugsweise 5 bis 90, am bevorzugtesten 20 bis 85 Gewichts% (basierend auf der Gesamtmischung) an Isomeren von trans/trans konjugierter Linolsäure (=tt-CLA) enthält, zur Herstellung eines Nahrungsmittels oder Nahrungsmittelzusatzes mit anti-inflammatorischen Eigenschaften eingesetzt wird.

2. Verwendung einer Mischung gemäss Anspruch 1, worin die Mischung, die die tt-CLA enthält, zur Herstellung eines Nahrungsmittelzusatzes in eingekapselter Form eingesetzt wird.

3. Verwendung einer Mischung gemäss Anspruch 1 und 2, worin die Mischung, die die tt-CLA-Isomere enthält, einen mit $^{13}$C-Magnetresonanztechnik gemessenen Gehalt an tt-CLA von 5 bis 90 Gewichts% (der Gesamtmischung) aufweist, wobei ebenfalls die CLA-Isomeren cis$^9$trans$^{11}$ und trans$^{10}$cis$^{12}$ in einem Gewichtsverhältnis von (Gesamt t$^9$t$^{11}$ + t$^{10}$t$^{12}$) : (c$^9$t$^{11}$ + t$^{10}$c$^{12}$) von mehr als 3:1, vorzugsweise mehr als 5:1, am bevorzugtesten von mehr als 7,5:1, vorliegen.

4. Verwendung einer Mischung gemäss Anspruch 1 bis 3, worin die tt-CLA-Isomeren entweder als freie Fettsäuren oder als Mono-, Di- und/oder Triglyceride oder als Alkylester eingesetzt werden.

5. Mischung mit anti-inflammatorischen Eigenschaften, enthaltend CLA-Isomere worin die Mischung mehr als 10 Gewichts%, vorzugsweise mehr als 25 Gewichts%, ganz besonders bevorzugt mehr als 50 Gewichts% CLA-Isomere enthält, von denen mehr als 55, vorzugsweise mehr als 60, besonders bevorzugt mehr als 65 Gewichts% (der gesamten CLA-Isomeren) tt-CLA-Isomere sind, gemessen mit $^{13}$C-Magnetresonanztechnik.

6. Mischung mit anti-inflammatorischen Eigenschaften gemäss Anspruch 5, worin die Mischung die tt-CLA-Isomeren t$^9$t$^{11}$ und t$^{10}$t$^{12}$ in einem Gewichtsverhältnis von t$^9$t$^{11}$:t$^{10}$t$^{12}$ von weniger als 2,3:1, vorzugsweise weniger als 1,8:1, am meisten bevorzugt weniger als 1,0:1 enthält, gemessen mit $^{13}$C-Magnetresonanztechnik.

**7.** Mischung gemäss Anspruch 5 oder 6, worin die Mischung auch $c^9t^{11}$- und $t^{10}c^{12}$-CLA-Isomere in einem Gewichtsverhältnis von (Gesamt $t^9t^{11} + t^{10}t^{12}$) : ($c^9t^{11} + t^{10}c^{12}$) von mehr als 3:1, vorzugsweise mehr als 5:1, am meisten bevorzugt mehr als 7,5:1 enthält, gemessen mit $^{13}$C-Magnetresonanztechnik.

**8.** Nahrungsmittel, vorzugsweise ausgewählt aus Konditoreiwaren, Brotaufstrichen, Saucen, Salatsaucen, Mayonnaise, Speiseeis, Teig, gebackene Backwaren, Füllungen und Cremen, enthaltend eine wirksame Menge einer Mischung, die 1 bis 100 Gewichts% tt-CLA enthält, vorzugsweise in Form eines Mono- und/oder Di- und/oder Triglycerids, am meisten bevorzugt enthaltend eine wirksame Menge einer Mischung gemäss Anspruch 5 bis 7.

**9.** Nahrungsmittelzusatz, enthaltend eine wirksame Menge einer Mischung, die 1 bis 100 Gewichts% tt-CLA enthält, vorzugsweise enthaltend eine wirksame Menge einer Mischung gemäss Anspruch 5 bis 7.

**10.** Nahrungsmittelzusatz gemäss Anspruch 9, worin die an tt-CLA reiche Mischung in einem in Nahrungsmittel zugelassenen Mittel zur Einkapselung eingekapselt ist.

**11.** Nahrungsmittelzusatz gemäss Anspruch 9 und 10, worin die tt-CLA enthaltende Mischung mit anderen Nahrungsmittelbestandteilen, ausgewählt aus den Vitaminen A, B, C, D, E und K, Mineralien, wie Kalzium, Kalium, Magnesium, Eisen, Kupfer, Zink, Selen und Antioxidantien, wie Tocopherole und Polyphenole, verstärkt ist

**12.** Verfahren zur Herstellung einer Mischung gemäss Anspruch 5 bis 7, worin

> i) eine Mischung mit einem Gesamtgehalt an unkonjugiertem C18:2 von mehr als 55 Gewichts% durch katalytische Behandlung mit einem Schwefel-Nickel-Katalysator bei einer Temperatur von 100 bis 180°C in Abwesenheit von Wasserstoff konjugiert und isomerisiert wird,
> ii) das in Schritt i) gebildete Produkt einer Lösungsmittel-Fraktionierung mit Aceton in einem Gewichtsverhältnis von Aceton zu CLA-Produkt von 10:1 bis 1:1 bei einer Temperatur von -25 bis -100°C unterworfen wird, worauf eine Stearin-(oder Kopf)-Fraktion als Produkt gemäß Anspruch 5 bis 6 isoliert wird.

**13.** Verfahren zur Anreicherung der tt-CLA-Isomeren in einem Gemisch, das verschiedene Isomere von konjugierter Linolsäure enthält, wobei ein Gemisch, das mindestens 1 Gewichts% an tt-CLA in Beimischung mit anderen CLA-Isomeren enthält, einer enzymatischen Umwandlung unterworfen wird, welche ausgewählt ist aus i) partieller Hydrolyse eines Glycerid-Gemisches oder eines Gemisches von Estern mit kurzkettigen Alkoholen, ii) partieller Veresterung eines Gemisches von freien Fettsäuren mit Glycerol oder einem Glycerid, iii) partieller Glycerolyse eines Glyceridgemisches und iv) partieller Veresterung eines Alkohols mit freien CLA-Isomeren, wobei ein Enzym verwendet wird, welches tt-CLA von anderen CLA-Isomeren unterscheiden kann, und danach das Reaktionsprodukt mit physikalischen Methoden abgetrennt wird, wobei eine Fraktion als Reaktionsprodukt abgetrennt wird, welche mit tt-CLA angereichert ist.

**14.** Verfahren nach Anspruch 13, worin das Enzym ausgewählt ist aus Lipase von Candida rugosa, Lipase QL, Lipase SL, Lipase OF, Lipase von Geotrichum candidum B, Lipozym IM, Lipozym M.

## Revendications

**1.** Utilisation d'une composition pour la production d'un produit alimentaire ou d'un complément alimentaire dans laquelle une composition comprenant de 1 à 100, de préférence de 5 à 90, plus préférentiellement de 20 à 85 % en poids (sur la base de la composition totale) d'isomères trans-trans d'acide linoléiquc conjugué (tt - CLA), tel que mesuré par résonance magnétique $^{13}$C, est utilisée pour la production d'un produit alimentaire ou d'un complément alimentaire ayant des propriétés anti-inflammatoires.

**2.** Utilisation d'une composition selon la revendication 1, dans laquelle la composition comprenant les isomères trans-trans d'acide linoléique conjugué est appliquée pour la production d'un complémenta alimentaire sous une forme encapsulée.

**3.** Utilisation d'une composition selon les revendications 1 et 2, dans laquelle la composition comprenant les isomères trans-trans d'acidylinoléique conjugué, contient tel que mesuré par les techniques de résonance magnétique $^{13}$C- de 5 à 90 % en poids (sur la base de la composition totale) d'isomères trans-trans d'acide linoléique conjugué, alors qu'également les isomères cis $^9$trans$^{11}$ et trans$^{10}$cis$^{12}$ d'acide linoléique conjugué sont présents dans un rapport en poids entre $t^9t^{11}$ + $t^{10}t^{12}$ total) et ($c^9t^{11}$ + $t^{10}c^{12}$) est supérieur à 3 pour 1, de préférence il est supérieur à 5 pour 1, de la façon la plus préférentielle il est supérieur à 7,5 pour 1.

4. Utilisation d'une composition selon les revendications 1 à 3, dans laquelle les isomères trans-trans d'acide linoléique conjugué sont appliqués soit sous la forme d'acides gras libres, ou de mono glycérides, de diglycérides et/ou de triglycérides, ou sous la forme d'alkyl esters.

5. Composition ayant des propriétés anti-inflammatoires comprenant des isomères d'acide linoléique conjugué, dans laquelle la composition comprend plus de 10 % en poids, de préférence plus de 25 % en poids, de la manière la plus préférentielle plus de 50 % en poids d'isomères d'acide linoléique conjugué parmi lesquels plus de 55, de préférence plus de 60, de la manière la plus préférés plus de 65 % en poids (sur la base du total des isomères d'acide linoléique conjugué) sont des isomètres trans-trans d'acide linoléique conjugué, tel que mesuré par les techniques de résonance magnétique $^{13}$C.

6. Composition ayant des propriétés anti-inflammatoires selon la revendication 5, dans laquelle la composition comprend les isomères trans-trans d'acide linoléique conjugué $t^9t^{11}$ et $t^{10}t^{12}$ dans un rapport en poids entre $t^9t^{11}$ et $t^{10}t^{12}$ inférieur à 2,3 pour 1, de préférence inférieur à 1,8 pour 1, plus préférentiellement inférieur à 1,0 pour 1 (tel que mesuré par les techniques de résonance magnétique $^{13}$C).

7. Composition selon les revendications 5 ou 6, dans laquelle la composition contient également des isomères $c^9t^{10}$ et $t^{10}c^{12}$ d'acide linoléique conjugué dans un rapport en poids entre ($t^9t^{11}$ + $t^{10}t^{12}$ total) et ($c^9t^{1\circ}$ + $t^{10}c^{12}$) supérieur à 3 pour 1, de préférence supérieur à 5 pour 1, plus préféreritiellement supérieur à 7,5 pour 1, tel que mesuré par la technologie de résonance magnétique $^{13}$C.

8. Produits alimentaires, de préférence sélectionné à partir du groupe constitué des produits de confiserie, des pâtes à tartiner, des sauces, des vinaigrettes, de la mayonnaise, des crèmes glacées, des pâtes, des produits de boulangerie cuits, des garnitures, des crèmes contenant une quantité effective d'une composition comprenant de 1 à 100 % en poids d'isomères trans-trans d'acide linoléique conjugué, de préférence sous la forme d'un monoglycéride, d'un diglycéride et/ou d'un triglycéride et, de la manière la plus préférée, contenant une quantité effective de la composition selon les revendications 5 à 7.

9. Compléments alimentaires contenant une quantité effective d'une composition comprenant de 1 à 100 % en poids d'isomères trans-trans d'acide linoléique conjugué, contenant de préférence une quantité effective d'une composition selon les revendications 5 à 7.

10. Compléments alimentaires selon la revendication 9, dans lesquels la composition riche en isomères trans-trans d'acide linoléique-conjugué est encapsulée dans un matériau d'encapsulation de qualité alimentaire,

11. Complément alimentaire selon les revendicafiom 9 à 10 dans lequel la composition comprenant les isomères trans-trans d'acide linoléique conjugué est fortifiée avec d'autres ingrédients alimentaires sélectionnés à partir du groupe constitué des vitamines A, B, C, D, E, K, des minéraux tels que le calcium, le potassium, le magnésium, le fer, le cuivre, le zinc; le sélénium, et les anti-oxydants tels que les tocophérols et les polyphénols.

12. Procédé de préparation d'une composition selon les revendications 5 à 7, dans lequel:

i) une composition contenant au total une teneur en $C_{18:2}$ non conjugué supérieure à 55 % en poids est conjuguée et isomérisée en la soumettant à un traitement catalytique avec un catalyseur soufre-nickel à une température de 100 à 180°C en l'absence d'hydrogène ;
ii) le produit formé dans le cadre de l'étape i) est soumis à un fractionnement au solvant en utilisant de l'acétone dans un rapport en poids entre l'acétone et le produit d'acide linoléique conjugué allant de 10 pour 1 à 1 pour 1 à une température allant de 25 à - 100°C, suite à quoi on isole une fraction (ou couche supérieure) de stéarine en tant que produit en accord avec la présente invention.

13. Procédé d'enrichissement d'un mélange comprenant différents isomères d'acide linoléique conjugué dans les isomères trans-trans d'acide linoléique conjugué, dans lequel un mélange comprenant au moins 1 % en poids d'isomères trans-trans d'acide linoléique conjugué en mélange avec d'autres isomères d'acide linoléique conjugué est soumis à une conversion enzymatique sélectionnée à partir du groupe constitué de i) l'hydrolyse partielle d'un mélange de glycéride ou d'un mélange, d'esters d'alcools à chaîne courte, ii) l'estérification partielle d'un mélange d'acides gras libres avec du glycérol ou avec un glycéride, iii) la glycérolyse partielle d'un mélange de glycéride et iv) l'estérification partielle d'un alcool avec des isomères d'acide linoléique conjugué libre, tout en utilisant une enzyme capable de discriminer les isomères trans-trans des autres isomères d'acide linoléique conjugué, suite à quoi le produit de réaction est séparé par des moyens physiques en séparant une fraction qui est enrichie en isomères trans-trans d'acide linoléique conjugué en tant que produit de réaction

14. Procédé selon la revendication 13, dans lequel l'enzyme est sélectionnée à partir du groups constitué de : la lipase *Candida rugosa*, la Lipase QL, la Lipase SL, la Lipase OF, la lipase B de *Geotrichum candidum*, le Lypozyme IM et le Lypozyme M.

Fig. 1 Effect of ttCLA on PGE2 levels.